(19) Europäisches Patentamt
European Patent Office
Office européen des brevets

(11) Publication number: 0 004 741 B1

# (12) EUROPEAN PATENT SPECIFICATION

(45) Date of publication of patent specification: 28.01.81

(21) Application number: 79300500.0

(22) Date of filing: 28.03.79

(51) Int. Cl.³: C 07 J 31/00, C 07 J 71/00, A 61 K 31/56, A 61 K 31/58 // C07J3/00

## (54) Thio etianic acid derivatives, their preparation and pharmaceutical use.

(30) Priority: 05.04.78 US 893388

(43) Date of publication of application: 17.10.79 Bulletin 79/21

(45) Publication of the grant of the patent: 28.01.81 Bulletin 81/4

(84) Designated Contracting States: BE CH DE FR GB IT NL SE

(56) References cited:
US-A-2 509 171
US-A-3 636 010
US-A-3 856 828
US-A-3 989 686

JOURNAL OF THE CHEMICAL SOCIETY, Serie C 1970 LONDON (GB) W. NAGATA and al.: "Hydrocyanation". Part IX Synthesis of β-Cyano-Aldehydes by Conjugate Hydrocyanation of Allylidene amines Followed by Hydrolysis, pages 2355-2364

(73) Proprietor: SYNTEX (U.S.A.) INC., 3401 Hillview Avenue, Palo Alto, California 94304 (US)

(72) Inventor: Edwards, John A., 5734 Arboretum Drive, Los Altos California 94022 (US)

(74) Representative: Armitage, Ian Michael et al, MEWBURN ELLIS & CO. 70/72 Chancery Lane, London WC2A 1AD (GB)

EP 0 004 741 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Thio etianic acid derivatives, their preparation and pharmaceutical use

This invention relates to novel derivatives of thio etianic acid (i.e. androstane 17β-thiocarboxylic acid) which are active anti-inflammatory agents in mammals. The invention further relates to pharmaceutically active compositions comprising a compound of the invention, and to a process for the preparation of these novel compounds.

Certain 3-oxoandrost-4-ene 17β-carboxylic acids which are substituted at the 9 position with chlorine or fluorine and at the 11 position with keto or hydroxy or chloro group are known. See for example U.S. 3,828,080 and 3,981,894 both to Phillipps et al. It is also known that 3-oxoandrost-4-ene 17β-carboxylic acids or esters thereof may be substituted at the 6α position with fluoro and optionally at the 9α position with a fluoro. See for example U.S. 3,636,010 and U.S. 4,093,721 to Phillipps.

It is also known from U.S. 3,989,686 to Phillipps et al of Glaxo that steroids of the formula

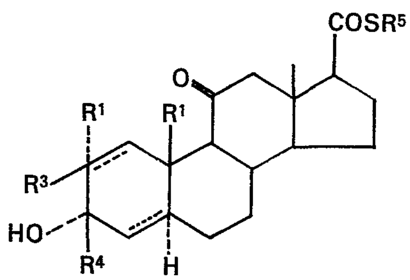

wherein
$R^1$ is H or $CH_3$;
$R^2$ is H or $CH_3$;
$R^3$ is H or, when $R^2$ is H, $C_{1-6}$ alkoxy, $C_{1-5}$ alkyl, thiocyanato or halogen;
$R^4$ is H or $CH_3$;
$R^5$ is $C_{1-6}$ alkyl optionally substituted by halo or $NR^6R^7$, where $R^6$ and $R^7$ are the same or different $C_{1-6}$ alkyl or $R^6$ and $R^7$ together with N are morpholino, thiamorpholine or morpholino substituted with $C_{1-6}$ alkyl; and
the dotted line in the "A" ring represents an optional double bond at these positions; are useful as anesthetics.

Methyl 3β-acetoxyallothiolcholonate and methyl 3β-acetoxy-etiothiochol-5-enate are also known. See, e.g., Jerger et al, Helv. Chem. Acta. 29, 684-92 (1947).

A heretofore unknown series of 3-oxoandrost-4-ene 17β-thiocarboxylates and derivatives thereof has been discovered and is disclosed herein. The 17β-thiocarboxylates exhibit topical anti-inflammatory activity and few adverse side effects.

One aspect of this invention is a compound chosen from those represented by the formula

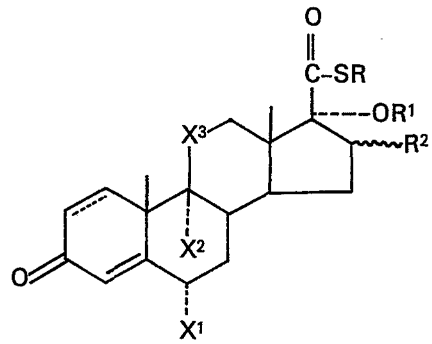 (I)

wherein
$X^1$ is hydrogen, fluoro, or chloro;
$X^2$ is hydrogen, fluoro, chloro or bromo;
$X^3$ is =C=O or 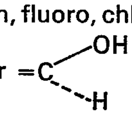 or may also be 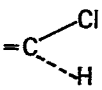
when $X^2$ is chloro;

R is alkyl of 1 to 6 carbon atoms, or phenyl or benzyl optionally substituted with a substituent on the phenyl ring which is alkyl of 1 to 4 carbon atoms, alkoxy of 1 to 4 carbon atoms or halo;
$R^1$ is hydrogen or alkanoyl of 2 to 6 carbon atoms when $R^2$ is hydrogen, α-methyl or β-methyl or $OR^1$ and $R^2$ together are 16α,17α-isopropylidenedioxy; and
the bond between C-1 and C-2 is a double or single bond.

Another aspect of this invention is a topical anti-inflammatory pharmaceutical composition which comprises a suitable pharmaceutical excipient in combination with an effective amount of a suitable compound chosen from those represented by Formula (I), above, wherein each of the substituents are as defined. Particularly valuable compounds in this composition are set forth hereafter.

Still another aspect of this invention is a compound of Formula I for use in treating an inflamed condition in mammals.

Still another aspect of this invention is a process for preparing a compound of this invention which process comprises treating an appropriate 17β-carboxylic acid or a corresponding reactive derivative thereof with a suitable base salt (preferably an alkali metal salt) of an appropriate alkanethiol, benzenethiol or phenylmethanethiol (RSH).

One subgroup of the compounds represented by Formula (I) are those wherein R is alkyl of 1-6 carbon atoms, phenyl or benzyl; $R^2$ is hydrogen, α-methyl or β-methyl when $R^1$ is alkanoyl of 2-6 carbon atoms or $OR^1$ and $R^2$ together are 16α,17α-isopropylidenedioxy; $X^1$ is fluoro or hydrogen; $X^2$ is fluoro, chloro or hydrogen; and $X^3$ is 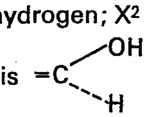 or may also be 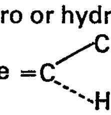 when $X^2$ is chloro. Of this

subgroup the compounds wherein $R^2$ is other than hydrogen, R is methyl or ethyl, $X^2$ is hydrogen or fluoro and $X^3$ is 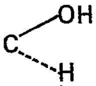 are preferred. Particularly preferred are those compounds wherein $X^1$ and $X^2$ are both fluoro. In each group or subgroup the compounds preferred are those with a double bond between C-1 and C-2.

Representatives examples of the compounds of this invention are set forth hereafter in the Examples.

In defining the compounds of this invention the term "alkyl" includes both straight chain and branched alkyl groups, thus "alkyl" of 1-6 carbons includes methyl, ethyl, n-propyl, isopropyl, n-butyl, isobutyl, t-butyl, pentyl, isoamyl, n-hexyl and the like. The phenyl and benzyl substituents may be substituted on the phenyl ring at the 2, 3 or 4-positions with one substituent such as alkoxy (e.g. methoxy, ethoxy, n-propoxy, t-butoxy and the like), alkyl of 1-4 carbons (e.g. methyl, ethyl, isopropyl, n-propyl, t-butyl, n-butyl, etc.), or a halo such as fluoro, chloro, bromo or iodo. Preferably the substitution is at the 2 or 4 positions.

The term "alkanoyl" refers to a radical of the formula 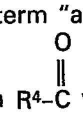 wherein $R^4$ is alkyl of 1-5 carbon atoms and includes, e.g., acetyl, propionyl, butyryl, valeryl, caproyl and the like.

In naming the compounds of this invention the substituents present on the androstane ring shall be included alphabetically and the compounds shall be alkyl (or phenyl or benzyl) 17β-carboxylates. For example, if in formula (I), above, $X^1$ is fluoro, $X^2$ is chloro, $X^3$ is 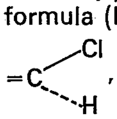, R is methyl, $R^1$ is acetoxy and $R^2$ is α-methyl the name is methyl 17α-acetoxy-9α, 11β-dichloro-6α-fluoro-16α-methyl-3-oxo-androsta-1,4-diene-17β-thiocarboxylate. If on the other hand, R is hydrogen but $X^1$, $X^2$, $X^3$, $X^4$, $R^1$ and $R^2$ are the same, the compound is named 17α-acetoxy-9α,11β-dichloro-6α-fluoro-16α-methyl-3-oxoandrosta-1,4-diene 17β-thiocarboxylic acid.

Another aspect of this invention is the preparation of the compounds of the invention. This can be done by reacting an appropriate androsta-1,4-diene 17β-carboxylic acid (or the corresponding 4-ene) or a reactive derivative, with an excess (e.g. about 1.1 to 5 molar equivalents based on the steroid) of alkali metal salt of a compound of the formula RSH where R is alkyl, benzyl or phenyl as defined hereinbefore. Representative alkali metal salts include, e.g., sodium methyl sulfide, sodium ethyl sulfide, sodium benzyl sulfide, sodium phenyl sulfide, potassium methyl sulfide, and the like. The alkali metal salt can be reacted directly with the reactive derivative of the 17β-carboxylic acid, or the salt can be formed in situ by mixing an alkali metal hydride, such as sodium hydride or potassium hydride, with an alkanethiol, benzenethiol or phenylmethanethiol. The thioesterification reaction readily takes place at temperatures of about 10° to 100°C (preferably at ambient temperatures of about 20-25°C) in a suitable inert solvent such as dimethylformamide, diethylformamide, dimethylacetamide, and the like. The reactive derivative of the 17β-carboxylic acid may be an acid chloride, but is preferably a mixed anhydride, such as the dialkyl phosphate ester prepared by reacting a dialkyl (1-4 carbons) chlorophosphate (e.g. diethyl chlorophosphate) with the appropriate 17β-carboxylic acid in an inert solvent such as tetrahydrofuran (THF) under an inert atmosphere (nitrogen).

The 17β-carboxylic acid starting materials are prepared by eliminating the 21 carbon atom from a suitable 21-hydroxy-3,20-dioxopregn-4-ene or pregna-1,4-diene. This is readily accomplished by any means known in the art such as using sodium hypobromite as taught in U.S. 2,769,822 or using sodium periodate to oxidize an appropriate 21-hydroxy pregnanes. Preferably, however, the elimination of the 21 carbon atom is carried out by using an alkali metal carbonate in alcohol and the presence of oxygen. In the latter case the reaction is carried out at room temperature and atmospheric pressure while the source of oxygen is preferably air. Generally the reaction will be completed within less than 24 hours with a constant stream of air being bubbled into a stirred reaction mixture.

Suitable 21-hydroxy-3,20-dioxopregn-4-enes or -pregna-1,4-dienes include known compounds such as corticosterone, hydrocortisone, prednisolone, β-methasone, dexamethasone, triamcinolone, paramethasone, fluocinolone, triamcinolone acetonide, fluocinolone acetonide, and the like. By following procedures generally known in the art steroids of a relatively simple structure can be converted to other structures as desired.

For example, the 6-fluoro starting steroids can be prepared from known steroids such as 17α-hydroxyprogesterone or hydrocortisone. The 6-fluoro group can be introduced by treating a 3-methoxy-pregna-3,5-diene (prepared by reacting a 3-keto-pregn-4-ene with triethyl orthoformate in methanol) with perchloryl fluoride in acetone-water 9:1.

Other 6-fluoro starting steroids employed in the present process to prepare the novel 17β-thiocarboxylic acid derivatives of this invention are described in the literature and in United States and foreign patents. For example, see U.S. Patents 2,983,737, 2,983,739, 3,053,838, 3,057,858, 3,124,251, 3,126,375, 3,201,391 and 3,248,389.

The 9α-fluoro, chloro or bromo group is introduced by treating a 9β,11β-oxido steroid with hydrogenfluoride, hydrogen chloride or hydrogen bromide respectively in an inert, nonaqueous, preferably anhydrous, solvent or mixture of such solvents. For example, see U.S. 3,211,758 to Tarkoey wherein a hydrogen fluoride/urea complex is employed. The 9β,11β-oxido steroid is prepared from the corresponding pregna-4,9(11)-diene (which is prepared by treating the corresponding 11β-hydroxy steroid with methane sulfonyl chloride in dimethylformamide in the presence of pyridine and a catalytic amount of sulfur trioxide) by treating the pregna-4,9(11)-diene with N-bromoacetamide and perchloric acid in dioxane or tetrahydrofuran, and then refluxing the resulting 9-bromo-11-hydroxy steroid with potassium acetate in acetone. The 9α,11β-dichloro groups are introduced by treating the corresponding pregna-4,9(11)-diene with chlorine gas in chloroform.

A 16-methyl group is introduced by treating the corresponding 20-keto-pregn-16-ene with methyl magnesium bromide in the presence of cuprous chloride in an ether such as tetrahydrofuran. The 20-keto-pregn-16-ene is prepared by preparing the 3,20-bis-semicarbazone of a 3,20-diketo-17α-hydroxy steroid, treating the semicarbazone with glacial acetic acid and acetic anhydride and then allowing the resulting product to react with aqueous pyruvic acid.

The 17α-hydroxy group is introduced in conjunction with the 16β-methyl group by first treating the corresponding 16-methyl-pregn-16-ene (which is prepared by treating the corresponding pregn-16-ene with diazomethane and then heating the resulting product to 180 °C) with hydrogen peroxide, in an aqueous basic media, then permitting the resulting 16,17-oxido-16-methyl steroid to react with hydrogen bromide in glacial acetic acid. The resulting 16,17-bromohydrin is hydrogenated with the use of a palladium catalyst to afford the corresponding 16β-methyl-17α-hydroxy derivative.

The 17α-hydroxy group is introduced in conjunction with the 16α-methyl by methods known in the art, such as the method described by Edwards et al in the Journal of the American Chemical Society 82, 2318-22, 1960. In this process an appropriate 21-substituted 16α-methyl-pregna-1,4-diene-3,20-dione is converted to 20-enol acetate by refluxing with acetic anhydride and freshly distilled acetyl chloride. The 20-enol acetate is recovered and reacted with monoperphthalic acid in ether and benzene to form the 17,20-epoxide which in turn is treated with methanol and aqueous potassium hydroxide to give the 16α-methyl-17α-hydroxy steroid which is isolated by means known in the art.

Another aspect of this invention is a process for preparing the compound of formula (I) wherein $R^1$ is alkanoyl of 2 to 6 carbon atoms and $X^1$, $X^2$, $X^3$, R, $R^2$ and the broken line between C-1 and C-2 are as defined in the broadest aspect of the invention. This process comprises esterifying the corresponding 17α-hydroxy compound. The esterification can be done by methods known in the art such as the methods set forth in U.S. Patent 3,828,080 issued August 6, 1974 to Phillips et al of Glaxo. Generally, the parent 17α-hydroxy compound is reacted with an appropriate carboxylic acid or a reactive derivative thereof such as an acid anhydride or acid chloride in the presence of a suitable acid catalyst and a solvent at temperatures of 20 to 100 °C. Suitable carboxylic acids and reactive derivatives include, e.g. acetic acid, propionic acid, butyric acid etc., and the corresponding anhydrides and acid chlorides. Solvents include non-hydroxylic solvents such as methylene chloride, chloroform, benzene and the like, while suitable acid catalysts include p-toluene-sulfonic acid, sulfosalicylic acid, perchloride acid, strongly acidic cation exchange resins, and the like.

The compounds of this invention are useful for the relief of inflamed conditions in mammals, and more specifically are useful for relieving inflammatory manifestations of corticosteroid responsive dermatoses. Initial approximation of anti-inflammatory activity is done by the following procedure of McKenzie, S.W. and Stoughton, R.B., "Method for Comparing Percutaneous Absorption of Steroids" Arch Dermat, 86, 608 (1962) or modifications thereof.

Generally, the inflammatory manifestation in mammals, particularly humans, is combatted by treating the afflicted mammal with a therapeutically effective amount of the novel steroids of this invention, that is an amount which results in improvement of the inflamed conditions.

Preferably the steroids are first formulated to prepare a suitable pharmaceutical formulation, as discussed hereinafter, which is then placed in contact with the afflicted area. An effective amount will depend upon the particular condition and the animal receiving the treatment but will vary between 0.001% to 10% by weight of the pharmaceutical composition and preferably will be between 0.02 and 1% by weight of the formulation. Using these levels in the formulation, a therapeutically effective and non-side effect producing amount, i.e. enough to effect an anti-inflammatory response, but not enough to adversely effect the recipient, is applied to the inflamed area.

The compounds of this invention not only have anti-inflammatory activity but also exhibit a low level of systemic activity, as measured by recognized laboratory assays. This allows for the application of an effective amount of the anti-inflammatory compounds with little adverse effect on the rest of the animal's system.

The novel steroids of this invention may be formulated with suitable pharmaceutical excipients known in the art to form particularly effective anti-inflammatory compositions which may be administered orally, nasally, rectally or, preferably, topically. Generally an effective amount of the steroid is about 0.001%w to about 10%w of the total formulated composition. The rest of the formulated composition will be about 90%w to about 99.999%w of suitable excipients which may include a pharmaceutically acceptable solvent and other pharmaceutically acceptable additives to form an effective pharmaceutical formulation. The composition is prepared by dissolving the active ingredient in the desired solvent and mixing with the other excipients as required.

A pharmaceutically acceptable solvent is one which is substantially non-toxic and non-irritating under the conditions used and may be readily formulated into any of the classical drug formulations such as powders, creams, ointments, lotions, gels, foams, suppositories, aerosols, solutions or the like. Particularly suitable solvents include water, glycerine, propylene carbonate, and a glycol such as 1,2-propylene diol (i.e. propylene glycol), 1,3-propylene diol, polyethylene glycol having a molecular weight of from 100 to 10,000, dipropylene glycol, etc.; and mixtures of the aforementioned with each other.

A topical cream may be prepared as a semi-solid emulsion of oil in water or water in oil. A cream base formulation by definition is an emulsion which is a two-phase system with one liquid (for example fats or oils) being dispersed as small globules in another substance (e.g., a glycol-water solvent phase) which may be employed as the primary solvent for the novel steroids therein, the cream formulation may contain fatty alcohols, surfactants, mineral oil or petrolatum and other typical pharmaceutical adjuvants such as antioxidants, antiseptics, or compatible adjuvants. A typical cream base formulation is given in the following table:

| | |
|---|---|
| Water/glycol mixture (15% or more glycol) | 50 - 99 parts by weight |
| Fatty alcohol | 1 - 20 |
| Non-ionic Surfactant | 0 - 10 |
| Mineral oil | 0 - 10 |
| Typical pharmaceutical adjuvants | 0 - 5 |
| Active Ingredients | 0.001 - 10 |

The fatty alcohol, non-ionic surfactant, and other adjuvants are discussed in U.S. 3,934,013 to Poulsen which is incorporated herein by reference.

The novel steroids of this invention may also be formulated as ointments. A "classical" ointment is a semisolid anhydrous composition which may contain mineral oil, white petrolatum, a suitable solvent such as a glycol and may include propylene carbonate and other pharmaceutically suitable additives such as surfactants, for example Span and Tween, or wool fat (lanolin), along with stabilizers such as antioxidants and other adjuvants as mentioned before. Following is an example of a typical "classical" ointment base:

| | |
|---|---|
| White petrolatum | 40 - 94 parts by weight |
| Mineral Oil | 5 - 20 |
| Glycol solvent | 1 - 15 |
| Surfactant | 0 - 10 |
| Stabilizer | 0 - 10 |
| Active Ingredients | 0.001 - 10.0 |

Other suitable ointment base formulations which employ propylene carbonate are described in U.S. patent 4,017,615 issued April 12, 1977 by Shastri et al entitled "Propylene Carbonate Ointment Vehicle" and U.S. 3,924,004 issued December 2, 1975 by Chang et al entitled "Fatty Alcohol-Propylene Carbonate-Glycol Solvent Cream Vehicle". As much of those applications as is pertinent is incorporated herein by reference. Following is a typical ointment base formulation containing propylene carbonate:

| | |
|---|---|
| Active Ingredients | 0.001 - 10.0 parts by weight |
| Propylene Carbonate | 1 - 10 |
| Solvent | 1 - 10 |
| Surfactant | 1 - 10 |
| White Petrolatum | 70 - 97 |

Surfactants, stabilizers, etc. are discussed in U.S. 3,934,013 and such discussion is incorporated herein by reference.

A suitable "non-classical" anhydrous, water washable "ointment type" base is described in U.S. Patent No. 3,952,930 to Katz and Neiman, and that patent is incorporated herein by reference. A representative composition of this invention utilizing such a base is as follows:

| | |
|---|---|
| Glycol solvent | 50 - 35 parts by weight |
| Fatty alcohol | 15 - 45 |
| Compatible plasticizer | 0 - 15 |
| Compatible coupling Agent | 0 - 15 |
| Penetrant | 0 - 20 |
| Active Ingredients | 0.001 - 10.0 |

Preparation 1

A process is set forth for preparing 16α-methyl-3-oxoandrosta-1,4-diene 17β-carboxylic acids substituted at the 9α-position with hydrogen, fluoro, chloro or bromo; at the 6α-position with hydrogen, fluoro or chloro; and at the 11β-position with hydroxy or chloro when 9α is chloro substituted.

A. Preparation of 6α,9α-difluoro-11β, 17α-dihydroxy-16α-methyl-3-oxoandrosta-1,4-diene 17β-carboxylic acid.

Thirty-five grams of flumethasone is mixed with 550 ml of methanol and 35 g of anhydrous potassium carbonate and stirred at room temperature and atmospheric pressure while a current of air is slowly bubbled through the solution for 22 hours. Methanol is added at intervals to maintain a constant volume. The reaction mixture is diluted with water to 1.5 l, then concentrated hydrochloric acid is added slowly to the mixture under magnetic stirring until a final pH of 2 is obtained. Methanol is eliminated under reduced pressure, and the resulting crystalline precipitate is collected by filtration, washed with water, and air dried to give 6α,9α-difluoro-11β,17α-dihydroxy-16α-methyl-3-oxoandrosta-1,4-diene-17β-carboxylic acid, melting point (m.p.) 289.5–290 °C.

B. By following the procedure set forth in part A of this example but substituting other appropriate starting materials the following compounds of this invention can be prepared:

9α,11β-dichloro-6α-fluoro-17α-hydroxy-16α-methyl-3-oxo androsta-1,4-diene 17β-carboxylic acid;

9α-chloro-6α-fluoro-11β,17α-dihydroxy-16α-methyl-3-oxo-androsta-1,4-diene 17β-carboxylic acid;

9α-bromo-6α-fluoro-11β,17α-dihydroxy-16α-methyl-3-oxo-androsta-1,4-diene 17β-carboxylic acid;

9α,11β-dichloro-17α-dihydroxy-16α-methyl-3-oxo-androsta-1,4-diene 17β-carboxylic acid;

11β,17α-dihydroxy-16α-methyl-3-oxo-androsta-1,4-diene 17β-carboxylic acid;

9α-chloro-11β,17α-dihydroxy-16α-methyl-3-oxo-androsta-1,4-diene 17β-carboxylic acid;

9α-bromo-11β,17α-dihydroxy-16α-methyl-3-oxo-androsta-1,4-diene 17β-carboxylic acid;

9α-fluoro-11β,17α-dihydroxy-16α-methyl-3-oxo-androsta-1,4-diene 17β-carboxylic acid;

6α-fluoro-11β,17α-dihydroxy-16α-methyl-3-oxo-androsta-1,4-diene 17β-carboxylic acid;

6α,9α-dichloro-11β,17α-dihydroxy-16α-methyl-3-oxo-androsta-1,4-diene 17β-carboxylic acid;

6α-chloro-9α-fluoro-11β,17α-dihydroxy-16α-methyl-3-oxo-androsta-1,4-diene 17β-carboxylic acid;

9α-bromo-6α-chloro-11β,17α-dihydroxy-16α-methyl-3-oxo-androsta-1,4-diene 17β-carboxylic acid;

6α-chloro-11β,17α-dihydroxy-16α-methyl-3-oxo-androsta-1,4-diene 17β-carboxylic acid; and

6α,9α-dichloro-11β,17α-dihydroxy-16α-methyl-3-oxo-androsta-1,4-diene 17β-carboxylic acid;

C. By following in principle the process of Part A but substituting other appropriate starting materials having a 17β-methyl group or being unsubstituted at 17 (e.g. β-methasone or hydrocortisone), other 17β-methyl or 17-unsubstituted starting materials are prepared.

D. Ten g of a compound prepared according to Parts A–C of this preparation is dissolved in 100 ml methanol. Fifty ml of anhydrous pyridine and 25 ml of propionic anhydride are added and the resulting mixture stirred until TLC shows the reaction is complete. The solution is cooled in an ice-water bath and slowly diluted with water up to 2 l. The resulting crystalline precipitate is collected by filtration and air dried to give the 17α-propionate. Other alkanoates are obtained by substituting other anhydrides for propionic anhydride.

Preparation 2

A process is set forth for preparing 16α,17α-isopropylidenedioxy-3-oxoandrosta-1,4-diene 17β-carboxylic acid substituted at 9α with hydrogen, fluoro, chloro or bromo; at 6α with hydrogen, fluoro or chloro; and at 11β with hydroxy or chloro when there is a 9α-chloro.

A. By following in principle the process of Preparation 1, Part A but substituting fluocinolone acetonide for flumethasone, one obtains 6α,9α-difluoro-11β-hydroxy-16α,17α-isopropylidenedioxy-3-oxoandrosta-1,4-diene 17β-carboxylic acid, m.p. 297–300 °C (with decomposition).

B. By following in principle the process of Part A of this preparation but substituting other 16α,17α-isopropylidenedioxypregna-1,4-diene-3,20-diones for fluocinolone acetonide, other 17β-carboxylic acids are obtained, for example

9α,11β-dichloro-6α-fluoro-16α,17α-isopropylidenedioxy-3-oxoandrosta-1,4-diene 17β-carboxylic acid;

9α-chloro-6α-fluoro-11β-hydroxy-16α,17α-isopropylidenedioxy-3-oxoandrosta-1,4-diene 17β-carboxylic acid;

6α,9α,11β-trichloro-16α,17α-isopropylidenedioxy-3-oxoandrosta-1,4-diene 17β-carboxylic acid; and the like.

Specific embodiments of the process of this invention are found in the following Examples which are given by way of illustration only and are not to be interpreted as limiting the scope of the claims appended hereto.

Example 1

A process is set forth for preparing alkyl, benzyl or phenyl 17α-alkanoyloxy-16α-methyl-3-oxoandrosta-1,4-diene 17β-thiocarboxylates of this invention which are substituted with hydrogen, fluoro or chloro at the 6α-position; with fluoro, chloro, bromo or hydrogen at the 9α-position; and 11β-hydroxy or 11β-chloro when there is a chloro at 9α.

A. Preparation of methyl 6α,9α-difluoro-11β-hydroxy-16α-methyl-3-oxo-17-propionyloxyandrosta-1,4-diene 17β-thiocarboxylate.

Six hundred (600) mg of 6α,9α-difluoro-11β-hydroxy-16α-methyl-3-oxo-17-propionyloxyandrosta-1,4-diene 17β-carboxylic acid, prepared in the manner set forth in Preparation 1,A, are mixed with 8 ml THF and 0.2 ml triethylamino (TEA) in a suitable reaction vessel and stirred at room temperature under $N_2$. Thereafter, .24 g of diethyl chlorophosphate (DCP: $(C_2H_5O)_2P(O)Cl$) in 8 ml THF is added over 13 minutes. Stirring is continued for about 6 hours (pH 6). Then, 0.04 ml TEA is added followed by .05 gm DCP in 3 ml THF. Stirring is continued for another 17.5 hours. The resulting precipitate is filtered, washed with 10 ml THF and the filtrates are combined. To the filtrate are then added 2.05 ml of a solution prepared previously which consists of 20 ml dimethylformamide (DMF), 0.758 g 75% sodium hydride and 0.86 g (1 ml) methanethiol. The resulting reaction mixture of the filtrates and the DMF solution is stirred for about 5.5 hours whereupon an additional 1 ml of the DMF solution is added and stirring is continued for another 1.5 hours.

The reaction mixture is then poured into 200 ml of ethyl acetate (EtOAc) which, in turn, is washed twice with 200 ml portions of water, washed with brine, dried for about 15 hours over sodium sulfate and filtered. The solvents are then removed under reduced pressure using a rotary evaporator to give 235 mg of residue which is recrystallized from acetone/hexane to give 54.3 mg of methyl 6α,9α-difluoro-11β-hydroxy-16α-methyl-17α-propionyloxyandrosta-1,4-diene 17β-thiocarboxylate, m.p. 305–308 °C (with decomposition).

B. By following in principle the procedure of Part A of this example but substituting other thiols such as ethanethiol, isopropanethiol, n-butan-

ethiol, benzenethiol, or phenylmethanethiol for methanethiol other compounds of this invention are prepared, namely

ethyl 6α,9α-difluoro-11β-hydroxy-16α-methyl-3-oxo-17α-propionyloxyandrosta-1,4-diene 17β-thiocarboxylate, m.p. 300 °C (with decomposition);

isopropyl 6α,9α-difluoro-11β-hydroxy-16α-methyl-3-oxo-17α-propionyloxyandrosta-1,4-diene 17β-thiocarboxylate, m.p. 286 °–289 °C;

n-butyl 6α,9α-difluoro-11β-hydroxy-16α-methyl-3-oxo-17α-propionyloxyandrosta-1,4-diene 17β-thiocarboxylate, m.p. 247 °–250 °C;

phenyl 6α,9α-difluoro-11β-hydroxy-16α-methyl-3-oxo-17α-propionyloxyandrosta-1,4-diene 17β-thiocarboxylate, m.p. 281 °–283 °C (with decomposition);

benzyl 6α,9α-difluoro-11β-hydroxy-16α-methyl-3-oxo-17α-propionyloxyandrosta-1,4-diene 17β-thiocarboxylate, m.p. 246–248 °C.

C. By following in principle the process of Part A of this example, but substituting other appropriate steroids for 6α,9α-difluoro-11β-hydroxy-16α-methyl-3-oxo-17-propionyloxyandrosta-1,4-diene 17β-carboxylic acid and other appropriate alkanethiols, benzenethiol or phenylmethanethiol for methanethiol, other compounds of this invention are obtained such as

methyl 6α,9α-difluoro-16α-methyl-3-oxo-17α-valerylandrosta-1,4-diene 17β-thiocarboxylate, m.p. 267–268 °C(d);

methyl 17α-butyryloxy 6α,9α-difluoro-11β-hydroxy-16α-methyl-3-oxo-17α-propionyloxyandrosta-1,4-diene 17β-thiocarboxylate, m.p. 276–280 °C(d);

methyl 17α-acetoxy-6α,9α-difluoro-11β-hydroxy-16α-methyl-3-oxoandrosta-1,4-diene 17β-thiocarboxylate, m.p. 285–290 °C(d);

methyl 17α-acetoxy 6α,9α-difluoro-16α-methyl-3-oxoandrosta-1,4-diene 17β-thiocarboxylate, m.p. +300 °C;

methyl 11β-hydroxy-16α-methyl-3-oxo-17α-propionyloxyandrosta-1,4-diene 17β-thiocarboxylate;

isopropyl 17α-acetoxy-9α-chloro-11β-hydroxy-16α-methyl-3-oxoandrosta-1,4-diene 17β-thiocarboxylate, m.p. 123–126 °C;

methyl-17α-butyryloxy-9α-fluoro-11β-hydroxy-16α-methyl-3-oxoandrosta-1,4-diene 17β-thiocarboxylate;

methyl 6α-fluoro-11β-hydroxy-16α-methyl-3-oxo-17α-propionyloxyandrosta-1,4-diene 17β-thiocarboxylate;

methyl 6α-chloro, 9α-fluoro-11β-hydroxy-16α-methyl-3-oxo-17α-propionyloxyandrosta-1,4-diene 17β-thiocarboxylate.

Example 2

A process is set forth for preparing alkyl, benzyl or phenyl 17α-hydroxy-16α-methyl-3-oxoandrosta-1,4-diene 17β-thiocarboxylates of this invention which are substituted at the 6α-position with fluoro, chloro or hydrogen; at the 9α-position with hydrogen, fluoro, chloro or bromo; and at the 11β-position with hydroxy or also chloro when there is a 9α-chloro.

A. Preparation of ethyl 6α,9α-difluoro-11β,17α-dihydroxy-16α-methyl-3-oxoandrosta-1,4-diene 17β-thiocarboxylate.

One hundred five (105) mg of 6α,9α-difluoro-11β,17α-dihydroxy-16α-methyl-3-oxoandrosta-1,4diene 17β-carboxylic acid are dissolved in 7 ml DMF and cooled to −10 °C. Eighty (80) mg carbonyl diimidazole (CDI) are dissolved in 30 ml DMF, and this solution is added to the DMF-acid solution under a nitrogen blanket. The resulting mixture is stirred for about 18 hours at −5 °C, and about 0.2 ml ethanethiol (EtSH) is added thereto. The reaction is stirred at −5 °C for an additional 16 hours. The reaction mixture is stored in the freezer for 2 weeks, the solvents removed under reduced pressure.

The residue is applied to a 1 meter X 0.75 TLC plate and developed twice with a mixture of 10% acetone/90% benzene. After recovery, the material is recrystallized from acetone/hexane to give 47 mg of ethyl 6α,9α-difluoro-11β,17α-dihydroxy-16α-methyl-3-oxoandrosta-1,4-diene 17β-thiocarboxylate, m.p. 235–239 °C (with decomposition).

B. Similarly, by following in principle the process of Part A of this example but substituting other alkanethiols, benzenethiol or phenylmethanethiol for ethanethiol, other compounds of this invention such as

methyl 6α,9α-difluoro-11β,17α-dihydroxy-16α-methyl-3-oxoandrosta-1,4-diene 17β-thiocarboxylate, m.p. 272–275 °C;

n-hexyl 6α,9α-difluoro-11β,17α-dihydroxy-16α-methyl-3-oxoandrosta-1,4-diene 17β-thiocarboxylate;

phenyl 6α,9α-difluoro-11β,17α-dihydroxy-16α-methyl-3-oxoandrosta-1,4-diene 17β-thiocarboxylate;

benzyl 6α,9α-difluoro-11β,17α-dihydroxy-16α-methyl-3-oxoandrosta-1,4-diene 17β-thiocarboxylate; and the like.

C. Similarly, by following in principle the process of Part A or Part B but substituting other appropriate 17β-carboxylic acid prepared in the manner set forth in Preparation 1 for 6α,9α-difluoro-11β,17α-dihydroxy-16α-methyl-3-oxoandrosta-1,4-diene 17β-carboxylic acid, other compounds of this invention are obtained such as

methyl 6α-fluoro-11β,17α-dihydroxy-16α-methyl-3-oxoandrosta-1,4-diene 17β-thiocarboxylate;

methyl 9α-fluoro-11β,17α-dihydroxy-16α-methyl-3-oxoandrosta-1,4-diene 17β-thiocarboxylate;

methyl 6α-fluoro-9α,11β-dichloro-17α-hydroxy-16α-methyl-3-oxoandrosta-1,4-diene 17β-thiocarboxylate; and the like.

Example 3

By following in principle the procedures set forth in Examples 1–2 but substituting the corresponding 16β-methyl steroid starting material for the 16α-methyl steroid starting material, the corresponding 16β-methyl steroids of this invention are obtained such as

methyl 17α-caproyloxy-9α-fluoro-11β-hydroxy-

16β-methyl-3-oxoandrosta-1,4-diene 17β-thiocarboxylate, m.p. 139–141 °C;
hexyl 17α-acetoxy-9α-fluoro-11β-hydroxy-16β-methyl-3-oxoandrosta-1,4-diene 17β-thiocarboxylate, m.p. 176–179 °C;
methyl 17α-acetoxy-9α,11β-dichloro-6α-fluoro-16β-methyl-3-oxoandrosta-1,4-diene 17β-thiocarboxylate;
and other corresponding 17α-alkanoyloxy derivatives along with other alkyl, phenyl or benzyl 17β-thiocarboxylates.

Example 4

By following in principle the procedures set forth in Example 1 but substituting the corresponding 16-unsubstituted steroid starting material for the 16α-methyl steroid starting material, the corresponding 16-unsubstituted steroids of this invention are obtained, such as
methyl 6α,9α-difluoro-11β,17α-dihydroxy-3-oxoandrosta-1,4-diene 17β-thiocarboxylate;
methyl 6α-fluoro-11β-hydroxy-3-oxo-17α-propionyloxyandrosta-1,4-diene 17β-thiocarboxylate;
hexyl 6α,9α-difluoro-11β-hydroxy-3-oxo-17α-propionyloxy-1,4-diene 17β-thiocaboxylate;
benzyl 6α,9α-difluoro-11β-hydroxy-3-oxo-17α-propionyloxy-1,4-dene 17β-thiocarboxylate;
and other corresponding alkyl, phenyl or benzyl 17βthiocarboxylates as well as the 17-α-alkanoyloxy derivatives.

Example 5

A process is set forth for preparing alkyl, benzyl or phenyl 16α, 17α-isopropylidenedioxy-3-oxoandrosta-1,4-diene 17β-thiocarboxylates of this invention which are substituted with hydrogen, fluoro or chloro at the 6α-position; with hydrogen, fluoro, chloro or bromo at the 9α-position; and with hydroxy at the 11β-position or also chloro at the 11β-position when there is a chloro at the 9α position.

A. Preparation of methyl 6α, 9α-difluoro-11β-hydroxy-16α, 17α-isopropylidenedioxy-3-oxo-androsta-1,4-diene 17β-thiocarboxylate.

Six hundred (600) mg of 6α, 9α-difluoro-11β-hydroxy-16α, 17α, isopropylidenedioxy-3-oxoandrosta-1,4-diene 17β-carboxylic acid (prepared in the manner described in Preparation 2, Part A) are mixed with 8 ml/THF and 0.21 ml TEA and stirred under nitrogen at room temperature. Three-tenths (0.3) of a ml of DCP in 11 ml THF is added to the steroid mixture over 6 minutes and the mixture is stirred at room temperature for about 17 hours, at which point, 5 drops of neat DCP are added. Stirring is continued for another 3.5, hours after which the precipitate in the reaction mixture is filtered and washed with 10 ml of THF. The filtrates are combined, and 3.15 ml of the solution of the methanethiol, sodium hydride, DMF as prepared in Example 1 is added thereto. The resulting mixture is stirred at room temperature under nitrogen for about 3.5 hours then poured into 300 ml of EtOAc which is then washed twice with 250 ml portions of water. The aqueous washes are extracted with 150 ml EtOAc, and the EtOAc solutions are combined, washed with brine and dried over sodium sulfate for about 15 hours in a refrigerator. The solvents are removed under reduced pressure by rotary evaporator to yield 546 mg of material which is recrystallized from acetone to give 309 mg of methyl 6α, 9α-difluoro-11β-hydroxy-16α, 17α-isopropylidenedioxy-3-oxoandrosta-1,4-diene 17β-thiocarboxylate, mp 299–301 °C (with decomposition).

B. Similarly, by substituting other alkanethiols, benzenethiol or phenylmethanethiol for methanethiol, and following in principle the process of Part A of this example, other compounds of this invention are prepared, such as
ethyl 6α,9α-difluoro-11β-hydroxy-16α, 17α-isopropylidenedioxy-3-oxoandrosta-1,4-diene 17β-thiocarboxylate, m.p. +300 °C(d);
isopropyl 6α,9α-difluoro-11β-hydroxy-16α, 17α-isopropylidenedioxy-3-oxoandrosta-1,4-diene 17β-thiocarboxylate;
n-butyl 6α,9α-difluoro-11β-hydroxy-16α,17α-isopropylidenedioxy-3-oxoandrosta-1,4-diene 17β-thiocarboxylate;
n-hexyl 6α,9α-difluoro-11β-hydroxy-16α,17α-isopropylidenedioxy-3-oxoandrosta-1,4-diene 17β-thiocarboxylate;
phenyl 6α,9α-difluoro-11β-hydroxy-16α,17α-isopropylenedioxy-3-oxoandrosta-1,4-diene 17β-thiocarboxylate;
benzyl 6α,9α-difluoro-11β-hydroxy-16α,17α-isopropylidenedioxy-3-oxoandrosta-1,4-diene 17β-thiocarboxylate; and the like.

C. Similarly by following in principle the procedure of Parts A and B of this example, but substituting other 17β-carboxylic acids prepared in the manner set forth in Preparation 2 for 6α,9α-difluoro-11β-hydroxy-16α,17α-isopropylidenedioxy-3-oxoandrosta-1,4-diene 17β-carboxylic acid, other compounds of this invention are prepared, such as
methyl 6α-fluoro-11β hydroxy-16α, 17α-isopropylidenedioxy-3-oxoandrosta-1,4-diene 17β-thiocarboxylate, m.p. +300 °C;
methyl 11β hydroxy-16α, 17α-isopropylidenedioxy-3-oxoandrosta-1,4-diene 17β-thiocarboxylate, m.p. +300 °C(d);
methyl 9α-fluoro-11β hydroxy-16α, 17α-isopropylidenedioxy-3-oxoandrosta-1,4-diene 17β-thiocarboxylate;
methyl 9α-chloro-6α-fluoro-11β hydroxy-16α,17α-isopropylidenedioxy-3-oxoandrosta-1,4-diene 17β-thiocarboxylate;
methyl 9α-bromo-6α-fluoro-11β hydroxy-16α,17α-isopropylidenedioxy-3-oxoandrosta-1,4-diene 17β-thiocarboxylate;
methyl 9α, 11β-dichloro-6α-fluoro-16α,17α-isopropylidenedioxy-3-oxoandrosta-1,4-diene 17β-thiocarboxylate;
n-butyl 9α,11β-dichloro-6α-fluoro-16α,17α-isopropylidenedioxy-3-oxoandrosta-1,4-diene 17β-thiocarboxylate;
phenyl 9α,11β-dichloro-6α-fluoro-16α,17α-isopropylidenedioxy-3-oxoandrosta-1,4-diene 17β-thiocarboxylate;
benzyl 9α,11β-dichloro-6α-fluoro-16α,17α-iso-

propylidenedioxy-3-oxoandrosta-1,4-diene 17β-thiocarboxylate; and the like.

Example 6

This example sets forth a process for preparing an 11-keto compound of this invention by oxidizing any of the 11β-hydroxy steroids set forth in Preparations 1–2 and converting the so-obtained compound to the 17β-thiocarboxylate according to the process of Examples 1–5.

One g of 6α,9α-difluoro-11β,17α-dihydroxy-16α-methyl-3-oxoandrosta-1,4-diene-17β-carboxylic acid is dissolved in 50 ml of acetone and treated at room temperature with Jones reagent (chromic anhydride in dilute sulfuric acid) dropwise until TLC indicates the absence of starting material. The mixture is treated with five drops of isopropyl alcohol to destroy any excess of reagent, then diluted with 50 ml of water and the mixture concentrated under vacuum under reduced pressure to give a crystalline material, namely 6α,9α-difluoro-17α-hydroxy-16α-methyl-3,11-dioxoandrosta-1,4-diene-17β-carboxylic acid. This compound, in turn, is reacted according to the process of Example 2 to give methyl 6α,9α-difluoro-17α-hydroxy-11-keto-16α-methyl-3-oxoandrosta-1,4-diene 17β-thiocarboxylate.

Example 7

This example sets forth a process for converting an androsta-1,4-diene of this invention to the corresponding androst-4-ene.

To a solution of 25 mg of tris-(triphenylphosphine) chlororhodium in 7 ml of benzene and 15 ml of ethanol, 244 mg methyl 6α,9α-difluoro-11β,17α-dihydroxy-16α-methyl-3-oxoandrosta-1,4-diene 17-thiocarboxylate are added and the resulting solution is stirred under hydrogen at room temperature at atmospheric pressure. After hydrogen uptake is complete the solution is evaporated to dryness and the residue is taken up in a mixture of petroleum ether and methylene chloride. The pure product is isolated by column chromatography on silica gel to give methyl 6α,9α-difluoro-11β,17α-dihydroxy-126α-methyl-3-oxoandrost-4-ene 17β-thiocarboxylate. Similarly, by substituting other androsta-1,4-dienes of this invention made according to examples 1–6 for the compound used above in this example, other corresponding androsta-4-enes of the invention are prepared such as methyl 6α-fluoro-11β-hydroxy 17α-propionyloxyandrost-1-ene 17β-thiocarboxylate, m.p. 210–212 °C.

Example 8 – $LD_{50}$

Six Swiss-Webster mice (Simonsen) each weighing about 25 grams, were injected subcutaneiously with a solution of methyl 6α,9α-difluoro-11β-hydroxy-16α-methyl-3-oxo-17α-propionyloxyandrosta-1,4-diene 17β-thiocarboxyate in carbomethoxycellulose having a concentration of 10 ml/kg. The dosage was 25 mg/kg or about .625 mg/mouse. The mice were observed daily for mortality for 21 days. No mice died. The $LD_{50}$ is, therefore, more than 25 mg/kg.

The same procedure was employed to find the $LD_{50}$ of methyl 6α,9α-difluoro-11-hydroxy-16α, 17α-isopropyledinedioxy-3-oxoandrost-1,4-diene. The $LD_{50}$ for this compound was found to be more than 25 mg/kg as well.

Example 9 – Biological Activity

This example sets forth data comparing the topical anti-inflammatory and thymolytic activities of compounds of this invention versus compounds known in the art. The topical anti-inflammatory activity potential for each compound was assayed using a modified Stoughton/McKenzie vaso-constriction assay in humans, as described by V.A. Place, J.G. Balasquez and K.H. Burdick in Arch. Dermat. 101, 531–537 (1970).

Eight normal adult human subjects were treated on four sites on each forearm by topical administration with alcoholic solutions containing $1\times10^{-5}$ and $1\times10^{-6}$ g/ml of each of the compounds to provide 64 total test sites for each compound in a series (32 for each concentration). Areas of the subjects' forearms were outlined by a rubber stamp grid coated with silicone grease, and 10 microliters of each solution were applied per 7×7 mm square site. After the preparations dried, the areas on each forearm are covered with Saran® wrap and the margins sealed with tape. The occlusive wrap is removed after 18 hours. Twenty-four hours after application, the presence or absence of vasoconstriction is noted by visual examination by two independent observers, and expressed as the number of sites responding (vasoconstriction) and is calculated as a percentage of the total number of sites. Also, the intensity of the vasoconstriction is cored on a 0, 1, 2 scale, 2 being the most intense reaction. Both scores are used in the constructing dose-response graphs according to methods set forth in an article by V.A. Place, infra. et al. The compounds of the invention (A and C) were compared directly with the compound of the art (B and D), respectively.

The compounds to be tested for thymolytic activity, including a hydrocortisone standard, were prepared in three or more concentrations by suspension in a sodium carboxymethyl-cellulose vehicle. Animals received the test materials by subcutaneous injection of 0.5 ml of the suspension on each of three successive days. Four hours following the final injection, the rats were sacrificed and the thymus gland of each animal removed and weighed. These weights are then employed to establish dose-response graphs by methods known in the art. Of the compounds tested only one pair showed measurable activity; the remaining compounds showed no activity in the dose range of hydrocortisone (the standard) and are therefore labelled inactive (less than 2 x hydrocortisone in potency).

In the data analysis shown (Table 1), each compound of this invention (A and C) bearing a thiomethyl ester function was compared directly with the closest oxygen analogue (B and D). Overall, the compounds show excellent topical potency with little or no systemic activity and distinct therapeutic advantage of the sulfur bearing compounds over the oxygen ester analogue is apparent. This superiority is surprising and unexpected.

Table 1

| | Rat Thymus (T) | Vasoconstriction (V) | Therapeutic Advantage |
|---|---|---|---|
| A. Methyl 6α,9α-difluoro-11β-hydroxy-3-oxo-16α-methyl-17α-propionyloxy-androstane-1,4-diene 17β-thiocarboxylate | 1 | ≥0.8 | ≥32 |
| B. Methyl 6α, 9α-difluoro-11β-hydroxy-3-oxo-16α methyl-17α-propionyloxy-androstane-1,4-diene 17β-carboxylate | 40 | 1 | |
| C. Methyl 6α,9α-difluoro-11β-hydroxy-3-oxo-16α,17α-isopropylidenedioxyandrosta-1,4-diene 17β-thiocarboxylate | Inactive | ≥10 | ≥10 |
| D. Methyl 6α,9α-difluoro-11β-hydroxy-3-oxo-16α,17α-isopropylidenedioxyandrosta-1,4-diene 17β-carboxylate | Inactive | 1 | |

In the case of Compound A vs Compound B, the therapeutic advantage of the compound of the invention was calculated according to the following equation:

$$\frac{V\,(\text{Comp A})}{T\,(\text{Comp A})} \div \frac{V\,(\text{Comp B})}{T\,(\text{Comp B})} = \geq \frac{0.8}{1.0} \div \frac{1}{40} = \geq 32$$

Because compound C is inactive in the rat thymus (T) test, the therapeutic advantage is given as ≥10.

Example 10

In this example a formulation is prepared of the following composition

| | % w/w |
|---|---|
| Methyl 6α,9α-difluoro-11β-hydroxy-16α-methyl-3-oxo-17α-propionyloxyandrosta-1,4-diene 17β-thiocarboxylate | 0.025 |
| Stearyl Alcohol | 30.0 |
| PEG 6000 | 5.0 |
| 1,2,6-Hexanetriol | 2.5 |
| Citric Acid Anhydrous, USP | 0.02 |
| Propylene Glycol, USP, q.s. | 100.0 |

The steroid is dissolved in 624.8 grams of propylene glycol at 90–95 °C. The latter is then mixed with the other ingredients at 80–85 °C to give the desired formulation.

**Claims**

1. A compound chosen from those represented by the formula

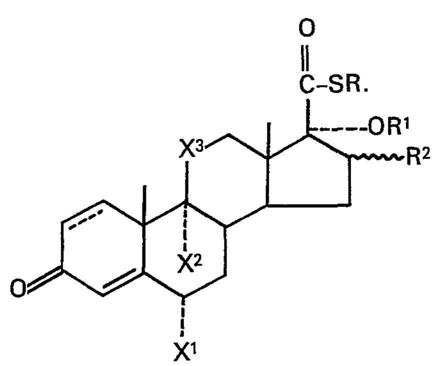

(I)

wherein

$X^1$ is hydrogen, fluoro, or chloro;

$X^2$ is hydrogen, fluoro, chloro or bromo;

$X^3$ is =C=0 or 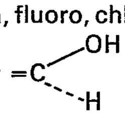 or may also be 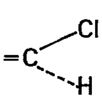

when $X^2$ is chloro;

R is alkyl of 1 to 6 carbon atoms, or phenyl or benzyl optionally substituted with a substituent on

the phenyl ring which is alkyl of 1 to 4 carbon atoms, alkoxy of 1 to 4 carbon atoms or halo;
$R^1$ is hydrogen or alkanoyl of 2 to 6 carbon atoms when $R^2$ is hydrogen, α-methyl or β-methyl or $OR^1$ and $R^2$ together are 16α,17α-isopropylidenedioxy; and the solid and broken lines between C-1 and C-2 represent a double or single bond.

2. A compound of Claim 1 wherein
R is alkyl of one to six carbon atoms, phenyl or benzyl;
$R^1$ is alkanoyl of two to six carbon atoms when $R^2$ is hydrogen, α-methyl or β-methyl or $OR^1$ and $R^2$ together are 16α,17α-isopropylidenedioxy;
$X^1$ is fluoro or hydrogen;
$X^2$ is fluoro, chloro or hydrogen; and
$X^3$ is 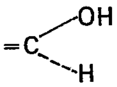 or may also be 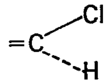 when $X^2$ is chloro.

3. A compound of Claim 2 wherein
R is methyl or ethyl;
$R^1$ is alkanoyl of two to six carbon atoms when $R^2$ is a α-methyl or β-methyl or $OR^1$ and $R^2$ together are 16α,17α-isopropylidenedioxy;
$X^1$is fluoro or hydrogen;
$X^2$ is fluoro or hydrogen; and
$X^3$ is 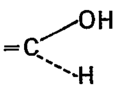 .

4. A compound of Claim 3 wherein $R^2$ is α-methyl and $X^1$ and $X^2$ are both fluoro.

5. The $\Delta^{1(2)}$-compound of Claim 4 wherein R is methyl and $R^1$ is propionyl, namely methyl 6α,9α-difluoro-11β-hydroxy-16α-methyl-3-oxo-17α-propionyloxyandrosta-1,4-diene 17β-thiocarboxylate.

6. The compound of Claim 1 wherein
$OR^1$ and $R^2$ together are 16α,17α-isopropylidenedioxy.

7. An anti-inflammatory pharmaceutical composition which comprises a therapeutically effective amount of the compound of Claim 1 in combination with at least one suitable pharmaceutical excipient.

8. A composition according to claim 7 suitable for topical application.

9. A compound of claim 1 for use in treating an inflamed condition in a mammal.

10. A process for preparing a compound of claim 1 which comprises
a) treating a compound of the formula

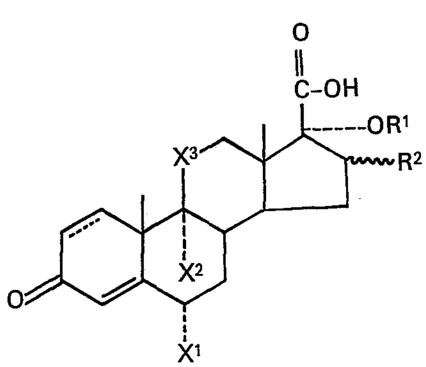

wherein $X^1$, $X^2$, $X^3$, $R^1$ and $R^2$ are as defined in claim 1 or a corresponding reactive derivative thereof with a suitable base salt of a compound represented by RSH wehrein R is as defined in claim 1; or
b) esterifying a compound of claim 1 in which $R^1$ is hydrogen to produce the corresponding compound in which $R^1$ is alkanoyl of 2 to 6 carbon atoms.

## Patentansprüche

1. Eine Verbindung der Formel:

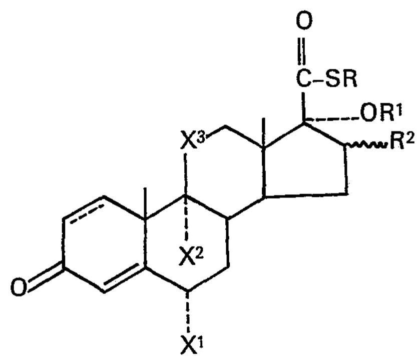

(I)

in welcher $X^1$ für Wasserstoff, Fluor oder Chlor steht;
$X^2$ für Wasserstoff, Fluor, Chlor oder Brom steht;
$X^3$ für =C=O oder 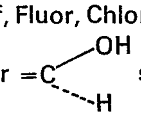 steht oder auch $X^3$ für 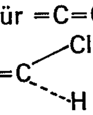 steht, wenn $X^2$ Chlor bedeutet;
R für $C_1$–$C_6$ Alkyl oder Phenyl oder Benzyl, gegebenenfalls mit einem Substituenten auf dem Phenylring, steht, der $C_1$–$C_4$ Alkyl, $C_1$–$C_4$ Alkoxy oder Halogen ist;
$R^1$ für Wasserstoff oder $C_2$–$C_6$ Alkanoyl steht, wenn $R^2$ für Wasserstoff, α-Methyl oder β-Methyl steht, oder $OR^1$ und $R^2$ zusammen 16α,17α-Isopropylidendioxy bedeuten,
und die durchgezogenen und gestrichelten Linien zwischen C-1 und C-2 eine Doppel- oder Einfachbindung bedeuten.

2. Verbindung nach Anspruch 1, dadurch gekennzeichnet, dass R für Alkyl mit 1 bis 6 Kohlenstoffatomen, Phenyl oder Benzyl steht;
$R^1$ Alkanoyl mit 2 bis 6 Kohlenstoffatomen bedeutet, wenn $R^2$ für Wasserstoff, α-Methyl- oder β-Methyl steht, oder $OR^1$ und $R^2$ gemeinsam für 16α,17α-Isopropylidendioxy stehen;
$X^1$ für Fluor oder Wasserstoff steht;
$X^2$ für Fluor, Chlor oder Wasserstoff steht; und
$X^3$ für 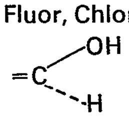 steht oder $X^3$ für 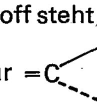 steht, wenn $X^2$ Chlor bedeutet.

3. Verbindung nach Anspruch 2, dadurch gekennzeichnet, dass
R für Methyl oder Äthyl steht;
$R^1$ Alkanoyl mit 2 bis 6 Kohlenstoffatomen bedeutet, wenn $R^2$ für α-Methyl oder β-Methyl steht,

oder $OR^1$ und $R^2$ gemeinsam für 16α,17α-Isopropylidendioxy stehen;
$X^1$ für Fluor oder Wasserstoff steht;
$X^2$ für Fluor oder Wasserstoff steht; und
$X^3$ für 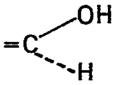 steht.

4. Verbindung nach Anspruch 3, dadurch gekennzeichnet, dass $R^2$ für α-Methyl steht und $X^1$ und $X^2$ jeweils Fluor bedeuten.

5. Die $\Delta^{1(2)}$-Verbindung nach Anspruch 4, in welcher R für Methyl und $R^1$ für Propionyl stehen, nämlich Methyl-6α,9α-difluor-11β-hydroxy-16α-methyl-3-oxo-017α-propionyloxyandrosta-1,4-dien-17β-thiocarboxylat.

6. Verbindung nach Anspruch 1, dadurch gekennzeichnet, das $OR^1$ und $R^2$ gemeinsam für 16α,17α-Isopropylidendioxy stehen.

7. Entzündungshemmendes pharmazeutisches Präparat, umfassend eine therapeutisch wirksame Menge einer Verbindung nach Anspruch 1 in Kombination mit mindestens einem geeigneten pharmazeutischen Streckmittel.

8. Präparat nach Anspruch 7, geeignet zur lokalen Anwendung.

9. Verbindung nach Anspruch 1 zur Verwendung in der Behandlung einer Entzündung bei Mensch und Tier.

10. Verfahren zur Herstellung einer Verbindung nach Anspruch 1, dadurch gekennzeichnet, dass man
a) eine Verbindung der Formel:

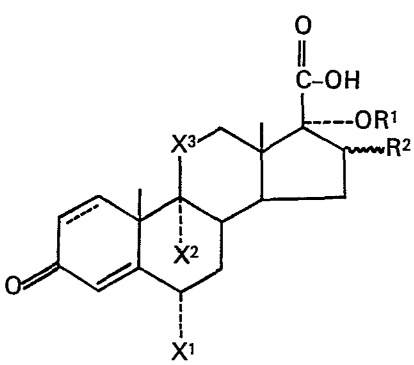

in welcher $X^1$, $X^2$, $X^3$, $R^1$ und $R^2$ die in Anspruch 1 genannte Bedeutung haben, oder ein entsprechendes, reaktionsfähiges Derivat derselben mit einem geeigneten basischen Salz einer Verbindung der Formel RSH, in welcher R die in Anspruch 1 genannte Bedeutung hat, behandelt; oder
b) eine Verbindung nach Anspruch 1, in welcher $R^1$ für Wasserstoff steht, zur Bildung der entsprechenden Verbindung, in welcher $R^1$ für eine Alkanoylgruppe mit 2 bis 6 Kohlenstoffatomen steht, verestert.

**Revendications**

1. Un composé choisi parmi ceux qui sont représentés par la formule:

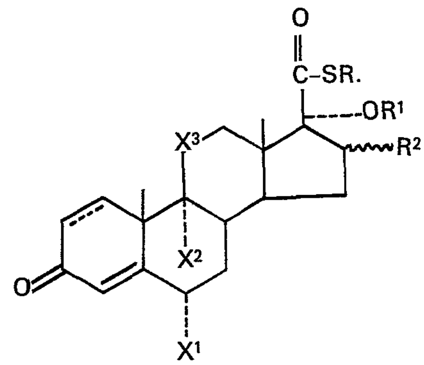

(I)

dans laquelle:
$X^1$ est un atome d'hydrogène, un radical fluoro ou un radical chloro;
$X^2$ est un atome d'hydrogène, un radical fluoro, un radical chloro ou un radical bromo;
$X^3$ est un radical =C=O ou 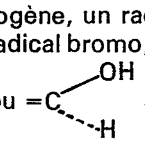 ou peut aussi être un radical 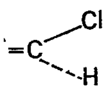 lorsque $X^2$ est un radical chloro;
R est un radical alkyle ayant 1 à 6 atomes de carbone ou un radical phényle ou benzyle portant éventuellement sur le noyau phényle un substituant qui est un radical alkyle ayant 1 à 4 atomes de carbone, alkoxy ayant 1 à 4 atomes de carbone ou halogéno;
$R^1$ est un atome d'hydrogène ou un radical alcanoyle ayant 2 à 6 atomes de carbone lorsque $R^2$ est un atome d'hydrogène, un radical α-méthyle ou un radical β-méthyle, ou bien $OR^1$ et $R^2$ forment ensemble un groupe 16α,17α-isopropylidènedioxy; et les liaisons en trait plein et en traits interrompus entre C-1 et C-2 représentent une double liaison ou une liaison simple.

2. Composé suivant la revendication 1, caractérisé en ce que:
R est un radical alkyle ayant 1 à 6 atomes de carbone, phényle ou benzyle;
$R^1$ est un radical alcanoyle ayant 2 à 6 atomes de carbone lorsque
$R^2$ est un atome d'hydrogène, un radical α-méthyle ou un radical β-méthyle, ou bien $OR^1$ et $R^2$ forment ensemble un groupe 16α,17α-isopropylidènedioxy;
$X^1$ est un radical fluoro ou un atome d'hydrogène;
$X^2$ est un radical fluoro ou chloro ou un atome d'hydrogène; et
$X^3$ est un radical 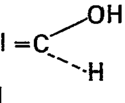 ou peut aussi être un radical 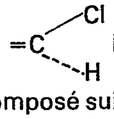 lorsque $X^2$ est un radical chloro.

3. Composé suivant la revendication 2, caractérisé en ce que
R est un radical méthyle ou éthyle;
$R^1$ est un radical alcanoyle ayant 2 à 6 atomes de carbone lorsque $R^2$ est un radical α-méthyle ou β-méthyle, ou bien $OR^1$ et $R^2$ forment ensemble

un groupe 16α,17α-isopropylidènedioxy;
$X^1$ est un radical fluoro ou un atome d'hydrogène;
$X^2$ est un radical fluoro ou un atome d'hydrogène; et

$X^3$ est un radical 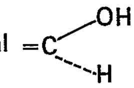 .

4. Un composé suivant la revendication 3, dans lequel $R^2$ est un radical α-méthyle et $X^1$ et $X^2$ sont chacun un radical fluoro.

5. Le composé $\Delta^{1(2)}$ suivant la revendication 4, dans lequel R est un radical méthyle et $R^1$ est un radical propionyle, à savoir le 6α,9α-difluoro-11β-hydroxy-16α-méthyl-3-oxo-17α-propionyloxy-androsta-1,4-diène-17β-thiocarboxylate de méthyle.

6. Le composé de la revendication 1, dans lequel $OR^1$ et $R^2$ forment ensemble un groupe 16α,17α-isopropylidènedioxy.

7. Une composition pharmaceutique anti-inflammatoire qui renferme une quantité à effet thérapeutique du composé suivant la revendication 1 en association avec au moins un excipient pharmaceutique convenable.

8. Une composition suivant la revendication 7, qui convient à l'application topique.

9. Un composé suivant la revendication 1, destiné à être utilisé dans le traitement d'un état inflammatoire chez un mammifère.

10. Procédé de préparation d'un composé suivant la revendication 1, qui consiste:
a) à traiter un composé de formule:

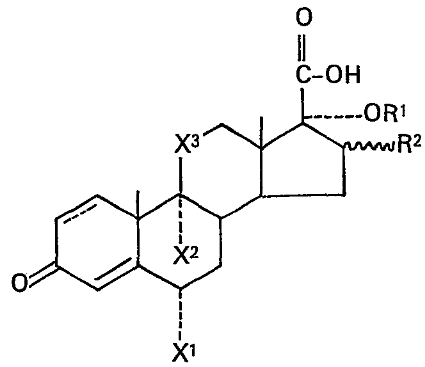

dans laquelle $X^1$, $X^2$, $X^3$, $R^1$ et $R^2$ ont les définitions données dans la revendication 1 ou un dérivé réactif correspondant de ce composé, avec un sel de base convenable d'un composé représenté par la formule RSH dans laquelle R a la définition donnée dans la revendication 1; ou
b) à estérifier un composé suivant la revendication 1, dans lequel $R^1$ est un atome d'hydrogène, pour produire le composé correspondant dans lequel $R^1$ est un radical alcanoyle ayant 2 à 6 atomes de carbone.